# EUROPEAN PATENT APPLICATION

(11) **EP 0 692 715 A1**
(43) Date of publication of application: **17.01.1996**
(21) Application number: 94110795.5
(22) Date of filing: 12.07.1994
(51) Int. Cl.: G01N 33/573, C07K 16/40

(54) **Method of assaying for fructose-1,6-biphosphatase and diagnostic test therefrom**

(71) Applicant: BAYER AG, D-51368 Leverkusen (DE)
(72) Inventor: Guder, Walter G. Prof.Dr.med., Englschalkinger Str.77 D-81925 München (DE); Hofmann, Walter Dr. med., Englschalkinger Str.77 D-81925 München (DE)

(57) **Abstract**

A method of assaying for fructose-1,6-biphosphatase in urine, and a method of diagnosing for acute kidney damage and a method of monitoring the nephrotoxic effects of a drug are disclosed.

## Description

### FIELD OF THE INVENTION

The present invention relates to the specific binding partner assay of urine for an enzyme, wherein presence of the enzyme is indicative of a diseased or damaged kidney. More particularly, the present invention relates to a specific binding partner method of assaying urine for the enzyme fructose-1,6-biphosphatase (FBP). The method is useful in a diagnostic test for acute kidney damage, and specifically for acute damage to the proximal tubule of the kidney.

### BACKGROUND OF THE INVENTION

FBP is one of four enzymes responsible for gluconeogenesis in the kidney. FBP dephosphorylates fructose-1,6-biphosphate to fructose-6-phosphate, which equilibrates to glucose-6-phosphate and eventually yields glucose. FBP also is present in the liver and has been found in human fetal brain tissue. FBP has an important role in gluconeogenesis in the liver.

The release of an enzyme (such as FBP) into a body fluid during various pathological conditions is known. Therefore, detection of such enzymes assist in the diagnosis of many disorders. For example, the assay for a particular enzymatic protein, or for enzyme activity, in serum and plasma has been used in diagnosing heart and liver diseases. The assay for a particular enzyme in diagnosing kidney damage and diseases is not customary however.

The kidneys are a target organ for the toxic action of various drugs and chemicals. The kidney therefore is subject to either chronic or acute damage. Nephropathy, or a kidney abnormality, due to the toxic action of drugs and chemicals often is manifested as proximal tubular necrosis.

In the kidney, FBP is reported to be localized in the proximal tubular epithelium (W.G. Guder et al. "Enzyme Distribution Along the Nephron", Kidney International, Vol. 26, pp. 101-111 (1984)). An increased permeability of renal epithelial cells can lead to release of proteins into the urine. Accordingly, investigators have detected elevated amounts of FBP in urine (which normally includes low amounts of FBP) because of leakage from damaged tubular cells.

The proximal tubule is an important component of the kidney because the proximal tubule reabsorbs about 65 to 70 percent of the sodium ions that pass through the glomerulus. Nephropathy often leads to a decreased glomerular filtration rate which is manifested by a rise in plasma creatinine concentration and a rise in plasma urea concentration, and by a fall in daily urine volume. However, significant kidney damage can occur before increased amounts of creatinine and urea are detectable. Accordingly, a sensitive test that reveals kidney damage in the early stages would be desirable.

Various investigators have studied the urinary excretion of enzymes in an effort to diagnose nephropathy of kidney graft rejection. For example, p. Kotanko et al., in "Urinary Enzyme Analysis in Renal Allograft Transplantation", Clinica Chemica Acta, 160, pp. 137-146 (1986), disclose that the presence of FBP, in conjunction (1986), disclose that the presence of FBP, in conjunction with the presence of three other specific enzymes, is an indication of kidney graft rejection. FBP and three other enzymes were assayed by measuring the enzymatic activity of the enzymes after administration of a drug having nephrotoxic side effects.

Other publications describing FBP and the presence of FBP in the kidney include:
W.G. Guder et al., "Biochemical Characterization of Individual Nephron Segments" Handbook of Physiology, The Kidney (E. Windhager, ed.), Oxford University Press, New York, pp. 2119-2164 (1992);
T. Biwas et al., "Fructose-1,6-bisphosphatase: Part III-Characterization of the Enzyme from Developing Human Fetal Liver", Ind. J. Biochem. Biophys., 22, pages 350-354 (1985);
T. Biwas et al., "Fructose-1,6-bisphosphatase: Part IV-Characterization of the Enzyme from Developing Human Fetal Brain", Ind. J. Biochem. Biophys., 22, pages 355-359 (1985); and
H. Schmid et al., "Specific Fructose-1,6-Bisphosphatase Activities in Microdissected Proximal Tubules of Human Kidneys in Function and Dysfunction", Molecular Nephrology, Proceedings of the 8th International Symposium, Dubrovnik, Yugoslavia, October 5-8, 1986, Z. Kovacevic et al. (Ed.), pages 339-345.

A need still exists however for a sensitive assay for FBP in urine, wherein assay results can be correlated to kidney damage, and particularly to acute damage to the proximal tubule. Such an assay and diagnostic method are important for individuals being treated with aminoglycosides or other drugs that are known to cause kidney damage. By monitoring the amount of FBP in urine, an individual then can monitor the effects of a nephrotoxic drug, like an aminoglycoside, on the kidney, and adjust dosages and treatment schedules accordingly.

### SUMMARY OF THE INVENTION

The present invention is directed to a specific binding partner method of assaying urine for a predetermined protein, and specifically, for a predetermined enzyme. In particular, the present invention is directed to the specific binding partner assay of urine for FBP. The presence of FBP in urine is an indicator of acute kidney damage, and particularly acute damage of the proximal tubule.

Previously, an increased creatinine concentration in plasma has been used to diagnose kidney damage. However, in the case of an acutely-damaged kidney, FBP is present in urine before an increased creatinine concentration in plasma is detectable. Therefore, an assay of urine for FBP provides an early detection test for acute kidney damage.

Accordingly, one aspect of the present invention is to provide a method of detecting acute tubular damage to the kidneys before conventional manifestations of kidney damage are apparent. Another aspect of the present invention is to provide a method of assaying urine specifically for FBP, and correlating the amount of FBP in the urine to acute damage to the proximal tubule of the kidney.

Another aspect of the present invention is to assay urine for FBP by a specific binding partner method, as opposed to an enzymatic method. The specific binding partner assay for FBP is more sensitive than an enzymatic activity assay. The specific binding partner assay also is independent of enzymatic inhibitors present in urine, thereby providing a more accurate assay for FBP.

The above and other aspects and advantages of the present invention will become apparent from the following detailed description of the invention.

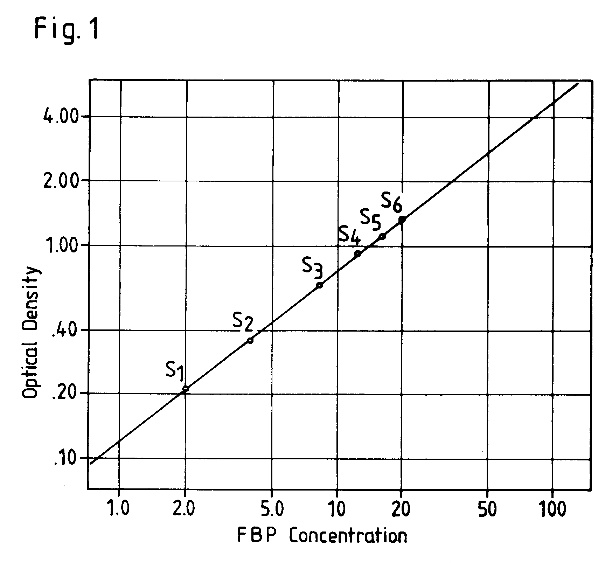

### BRIEF DESCRIPTION OF THE DRAWING

The figure is a plot of FBP concentration versus optical density for a series of FBP assays using a specific binding partner assay.

### DETAILED DESCRIPTION OF THE PREFERRED EMBODIMENTS

FBP is present in different tissues, including the kidney. Specifically, FBP is present in the proximal tubule of the kidney. FBP is present in urine of a healthy individual in low concentrations, but acute damage to the proximal tubule of the kidney can result in a release of FBP into the urine thereby increasing urinary FBP concentration.

For example, various drugs can damage the kidney and therefore the dosage of such drugs is monitored carefully. The damage from such drugs can be monitored by assaying serum or plasma for creatinine. Typically, creatinine concentration increases with the kidney damage.

The assay for creatinine has disadvantages because although an increased creatinine concentration is an indicator of kidney damage, the assay does not indicate where kidney damage has occurred, or whether the damage is acute or chronic. The present method overcomes these disadvantages, and provides an early detection of acute damage to the proximal tubule of the kidney by the specific binding partner assay of urine for FBP.

To illustrate that increased amounts of FBP in urine is an indicator of acute damage to the proximal tubules, the following tests were performed on a reference population and on individuals suffering from various kidney diseases. In each test, the urinary FBP concentration was measured using a specific binding partner assay for FBP, and more particularly a sandwich immunoassay described in detail hereinafter. First, 120 urine samples from 30 different individuals were assayed for FBP. This reference population of healthy individuals had a urinary FBP concentration in the range of less than about 10 to about 50 µg FBP/g creatinine (micrograms of FBP per gram of creatinine).

The concentration of FBP in urine is expressed as µg FBP/g creatinine to make the assay more independent of total urine concentration. The amount of an analyte excreted in urine often is not reported as a simple concentration because the concentration of an analyte varies with the concentration of the urine as a whole. Therefore, the concentration of an analyte is expressed as a ratio of the concentration of the analyte to the concentration of creatinine in the urine sample.

Creatinine is selected as the second analyte in the ratio because creatinine is excreted at an essentially constant rate, which is directly proportional to the muscle mass of an individual. Therefore, a ratio of analyte concentration to creatinine concentration provides a more accurate assay because the analyte assay is more independent of the effects of very dilute or very concentrated urine samples. The concentration of analyte and the concentration of creatinine both are inversely proportional to the amount of water in the urine sample. Since creatinine excretion is essentially constant, the effects of water in the urine sample are negated. The concentration of the analyte therefore is normalized.

Typically, one liter of normal urine includes one gram of creatinine. Therefore, the reference range of less than about 10 to about 50 µg FBP/g creatinine for healthy individuals is essentially equal to a reference range of less than about 10 to about 50 µg FBP/L (liter) of urine.

The reference range of less than about 10 to about 50 µg FBP/g creatinine, i.e., less than about 10 to about 50 µg FBP/L urine, was compared to the urinary FBP concentration of six individuals being treated with an aminoglycoside (i.e., gentamicin). Gentamicin is known to be nephrotoxic, and individuals treated with gentamicin exhibit an increased urinary FBP concentration. During the course of the gentamicin treatment (1-5 days), the median urinary FBP concentration of the six individuals increased to 190 µg FBP/g creatinine. The highest concentration was 571 µg FBP/g creatinine. The FBP concentrations were determined by the present specific binding partner assay for FBP.

In contrast to the acute kidney damage caused by gentamicin, one hundred thirty-four individuals suffering from chronic glomerulonephritis or a tubulo-interstitial nephropathy exhibited a urinary FBP concentration below 20 µg FBP/g creatinine. In addition, three individuals having an acute kidney transplant rejection exhibited urinary FBP concentrations below 50 µg FBP/g creatinine throughout the investigation.

The urinary FBP concentration therefore was in the normal range of less than 10 to about 50 µg FBP/g creatinine (i.e., less than about 10 to about 50 µg FBP/L urine) for healthy individuals, individuals having chronic kidney ailments and individuals having an acute transplant rejection. However, individuals suffering from acute kidney damage (e.g., individuals treated with gentamicin) exhibited up to a 10 fold increase in urinary FBP concentration. Therefore, measurement of urinary FBP concentration is an indicator of acute damage to the proximal tubule of the kidney, the site in the kidney where FBP is located.

The presence of FBP in the proximal tubule is known. However, until the method of the present invention, the correlation between an increased urinary FBP concentration and acute kidney damage has not been made. In addition, previous methods of assaying for FBP measured the enzymatic activity of the test sample, and correlated the enzymatic activity to FBP concentration. Often, the enzymatic activity measurements were inaccurate because a portion of the FBP in urine can be damaged and therefore is enzymatically inactive. In addition, enzymatic inhibitors can be present in the urine. Accordingly, measuring enzymatic activity yields erroneously low values for urinary FBP concentration. However, the present specific binding partner method provides an accurate and sensitive assay of urine for FBP because damaged and undamaged FBP is assayed by a specific binding partner for urinary FBP, and the specific binding partner is unaffected by enzymatic inhibitors.

The specific binding partner assay for FBP was performed by first generating a specific binding partner for FBP by standard procedures known to those skilled in the art. An exemplary procedure is illustrated "Methods in Enzymatic Analysis", H.U. Bergmeyer (Ed.), Vol IX, pp. 15-37 (1985).

In general, two rabbits were subcutaneously immunized with a solution of 50 µg (microgram) of FBP (the antigen) mixed with 500 µL (microliters) Alugel and Freund's adjuvant. Booster injections of the FBP antigen solution were administered to the rabbits once a week for a period of 5 weeks after the first immunization.

More particularly, pure kidney FBP (50 µg) was emulsified in Freund's adjuvant (ratio 1:1 v/v) with the addition of 1M sodium chloride solution, as needed, to promote emulsification and provide a final volume of 500 µL. The resulting emulsion was injected into the rabbits, subcutaneously, at multiple sites. Booster injections were performed weekly, for four consecutive weeks, with a solution containing 20 µg FBP in 500 µL aqueous sodium chloride. Blood was collected from the rabbits one week following the final (i.e., fifth) injection.

The specific anti-FBP serum IgG fraction was isolated by affinity chromatography on a protein A-sepharose Cl 4B affinity column. The protein A-sepharose Cl 4B column was equilibrated overnight with PBS buffer having a pH of 7.2. Then, the rabbit serum (8 mL) was pumped over the protein A-sepharose Cl 4B column overnight. The column then was washed with PBS buffer until no protein was detected in the eluted fractions. The IgG fraction was eluted using a 0.1M glycine hrydrochloride buffer having a pH of 3.0.

A sandwich immunoassay for FBP then was developed. The specific antibodies against FBP were isolated by immunoaffinity chromatography by CN-Br activated sepharose, which was coupled with pure FBP. The isolated anti-FBP IgG fraction was labelled with Biotin by the method disclosed in P.V. Subba Rao et al., "An Avidin-Biotin MicroELISA for Rapid Measurement of Total and Allergen Specific Human IgE", J. Immunol.Methods, 57, pp. 71-85 (1983). Concentrations of FBP were measured with a calibration curve.

The specific binding partner method detected and measured FBP over the concentration range of about 2 to about 20 µg FBP/L urine. The figure is a plot of FBP concentration versus optical density for the assay of urine samples containing 2 µg FBP/L through 20 µg FBP/L urine. The plot illustrates the excellent linear response between FBP concentration and optical density provided by the method of the present invention.

It should be understood however that the present specific binding partner method is useful for assaying urines having a FBP concentration outside of the range of about 2 to about 20 µg FBP/L of urine sample. The linear response illustrated in the figure extends below about 2 µg FBP/L of urine and above about 20 µg FBP/L of urine. Furthermore, if necessary and as known to persons skilled in the art, urine samples having a FBP concentration above about 20 µg FBP/L of urine, and up to about 2x10⁴ µg FBP/L of urine, can be diluted with water at a sufficient ratio of urine sample to water, such as about 1:1 to about 1:1000, to provide a solution having an FBP concentration of about 2 to about 20 µg FBP/L of urine. Assay results then can be corrected by the dilution factor to provide the true FBP concentration of the urine sample. Therefore, a urine sample containing up to 2x10⁴ µg FBP/L urine, when properly diluted, can be assayed for FBP concentration by the method of the present invention.

Normal urine has a FBP concentration of less than about 10 to about 50 µg FBP/L urine. Individuals treated with aminoglycosides exhibited increased median urine FBP concentration of about 190 µg FBP/g creatinine. Therefore, it is estimated that a FBP concentration above about 50 µg FBP/L urine indicates the onset of acute damage to the proximal tubule of the kidney.

The present specific binding partner method also is analytically precise, as demonstrated by the following test data from assays for FBP on urine samples containing 12 µg FBP/L urine.

| | Intrassay Variation¹⁾ | Interassay Variation²⁾ |
|---|---|---|
| Concentration (µg/L) | 12 | 12 |
| Measurement(n)³⁾ | 12 | 13 |
| CV(%)⁴⁾ | 10 | 12 |
| | | |

| | | |
|---|---|---|
| 1) measurements within a series of 12 assays; | | |
| 2) measurements from day to day over a period of eleven days; | | |
| 3) n = number of tests; and | | |
| 4) CV = standard deviation/mean x 100%. | | |

The increased urinary FBP concentration in an individual suffering from acute kidney damage as opposed to chronic kidney damage indicates that FBP is an indicator of acute tubular damage. In addition, the present specific binding partner assay for FBP provides a more sensitive assay for acute kidney damage than prior methods that measure serum creatinine levels, and a more accurate assay for FBP than prior enzymatic detection methods. In addition, the assay for urinary FBP provides an earlier indication of acute tubular damage.

Obviously, many modifications and variations of the invention as hereinbefore set forth can be made without departing from the spirit and scope thereof and therefore only such limitations should be imposed as are indicated by the appended claims.

## Claims

1. A method of assaying a urine sample for fructose-1,6-biphosphatase comprising:
(a) incorporating a specific binding partner for fructose-1,6-biphosphatase into a device in a manner such that the device is capable of exhibiting a detectable and measurable response to interactions between fructose-1,6-biphosphatase and the specific binding partner;
(b) contacting the device with the urine sample;
(c) examining the device for a detectable response; and
(d) correlating the detectable response in the device to the amount of fructose-1,6-biphosphatase in the urine sample.

2. The method of claim 1 wherein the urine sample is undiluted.

3. The method of claim 1 wherein the urine sample is diluted.

4. The method of claim 1 wherein the fructose-1,6-biphosphatase is present in the urine sample in an amount of up to about 2x10⁴ µg FBP/L of urine.

5. The method of claim 2 wherein the fructose-1,6-biphosphatase is present in the undiluted urine sample in an amount of about 2 to about 20 µg FBP/L of urine.

6. The method of claim 3 wherein the fructose-1,6-biphosphatase is present in the diluted urine sample in an amount of about 2 to about 20 µg FBP/L of urine.

7. A method of diagnosing acute kidney damage comprising:
(a) contacting a urine sample with a device capable of undergoing a detectable and measurable response to fructose-1,6-biphosphatase present in the urine sample, said device having incorporated therein a specific binding partner for fructose-1,6-biphosphatase;
(b) examining the device for a response; and
(c) correlating the response to the amount of fructose-1,6-biphosphatase the urine sample.

8. The method of claim 7 wherein the fructose-1,6-biphosphatase in an amount of above about 50 µg FBP/L of urine is indicative of acute kidney damage.

9. The method of claim 7 wherein the acute damage is in a proximal tubule of the kidney.

10. A method of monitoring the nephrotoxic effects of a drug comprising:
(a) contacting a urine sample with a device capable of undergoing a detectable and measurable response to fructose-1,6-biphosphatase present in the urine sample, said device having incorporated therein a specific binding partner for fructose-1,6-biphosphatase;
(b) examining the device for a response;
(c) correlating the response to the amount of fructose-1,6-biphosphatase in the urine sample; and
(d) comparing the amount of fructose-1,6-biphosphatase to the amount of fructose-1,6-biphosphatase in a previous urine sample, wherein an increased amount of fructose-1,6-biphosphatase in the urine is indicative of the nephrotoxic effects of the drug.
